(19) Europäisches Patentamt
European Patent Office
Office européen des brevets

(11) **EP 2 683 256 B1**

(12) **FASCICULE DE BREVET EUROPEEN**

(45) Date de publication et mention de la délivrance du brevet:
**19.07.2017 Bulletin 2017/29**

(51) Int Cl.:
*A23L 33/20* *(2016.01)*    *A23L 33/21* *(2016.01)*
*A61K 31/715* *(2006.01)*

(21) Numéro de dépôt: **12714751.0**

(22) Date de dépôt: **07.03.2012**

(86) Numéro de dépôt international:
**PCT/FR2012/050472**

(87) Numéro de publication internationale:
**WO 2012/120236 (13.09.2012 Gazette 2012/37)**

(54) **COMPOSITION NUTRACEUTIQUE POUR LIMITER L'ABSORPTION DE LIPIDES ALIMENTAIRES ET POUR INDUIRE UNE PERTE DE POIDS COMPRENANT COMME AGENT ACTIF AU MOINS UN EXTRAIT DE CAROTTE**

NÄHRENGÄNZUNGSMITTEL FÜR DIE VERMINDERUNG DER ABSORPTION DER FETTE UND FÜR GEWICHTSABNEHMEN, ENTHALTEND WENIGSTENS EINEN KAROTTENEXTRAKT

NUTRACEUTICAL COMPOSITION FOR LOWERING THE ABSORPTION OF DIETARY LIPIDS ET INDUCING WEIGHT LOSS, COMPRISING AT LEAST ONE CARROT EXTRACT AS ACTIVE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **08.03.2011 FR 1151890**

(43) Date de publication de la demande:
**15.01.2014 Bulletin 2014/03**

(73) Titulaire: **LAB ATTITUDE**
**13015 Marseille (FR)**

(72) Inventeur: **DIDDEN, Laurent**
**F-13190 Allauch (FR)**

(74) Mandataire: **Roman, Alexis**
**Cabinet Roman**
**35 rue Paradis**
**B.P. 30064**
**13484 Marseille Cedex 20 (FR)**

(56) Documents cités:
FR-A1- 2 894 777       JP-A- 2008 189 571
KR-A- 20040 095 949    US-A1- 2001 012 534
US-A1- 2010 074 969    US-B1- 6 413 545

- **HURSEL R ET AL: "Effects of green tea on weight loss and weight maintenance. A meta-analysis", APPETITE, ACADEMIC PRESS, NEW YORK, NY, US, vol. 52, no. 3, 1 juin 2009 (2009-06-01), page 838, XP026151769, ISSN: 0195-6663, DOI: 10.1016/J.APPET.2009.04.099 [extrait le 2009-06-01]**
- **CHOU SY, CHIEN PJ, CHAU CF: "Particle size reduction effectively enhances the cholesterol-lowering activities of carror insoluble fiber and cellulose", J. AGRIC. FOOD CHEM., vol. 56, 2008, pages 10994-10998, XP002660102,**

Remarques:
Le dossier contient des informations techniques présentées postérieurement au dépôt de la demande et ne figurant pas dans le présent fascicule.

EP 2 683 256 B1

**Description**

**Domaine technique de l'invention.**

La présente invention a pour objet une composition nutraceutique pour son utilisation pour limiter l'absorption de lipides alimentaires et pour induire une perte de poids comprenant comme agent actif au moins un extrait de carotte.
L'invention concerne également l'utilisation d'une telle composition en tant que complément alimentaire ou pour enrichir une boisson ou un produit alimentaire.
L'invention concerne encore une méthode non thérapeutique pour limiter l'absorption de lipides alimentaires et pour induire une perte de poids.

**État de la technique.**

Le surpoids se définit comme une accumulation anormale ou excessive de graisses dans le tissu adipeux. Le surpoids est un facteur de risque majeur pour un certain nombre de maladies chroniques, y compris le diabète, les maladies cardiovasculaires, le cancer.
Le principal indicateur de mesure du surpoids et de l'obésité est l'indice de masse corporelle (IMC). Ce dernier correspond au poids corporel en kilogrammes divisé par le carré de la taille en mètres d'une personne (unité = $kg/m^2$). Chez l'adulte, un IMC compris entre 18,5 et 24,9 est normal. Un IMC compris entre 25 et 29,9 correspond à un surpoids et un IMC de 30 et plus indique une obésité. Ces seuils servent de repères pour une évaluation individuelle, mais il est attesté que le risque de maladies chroniques augmente progressivement au-delà d'un IMC d'environ 22.
En raison du fait qu'il est difficile de se restreindre sur le plan alimentaire et de lutter contre la prise de calories indésirables, notamment de lipides, il s'est avéré souhaitable de pouvoir disposer d'une composition nutraceutique et d'un complément alimentaire intégrant une telle composition qui profitent aux personnes qui cherchent à perdre du poids pour améliorer leur santé ou leur bien-être physique, notamment aux personnes en surpoids ne présentant pas de troubles médicaux associées au surpoids ou à l'obésité, particulièrement aux personnes ayant un indice IMC compris entre environ 25 et environ 30. Une composition nutraceutique et un complément alimentaire intégrant une telle composition basés sur des produits naturels qui permettent d'agir d'une part sur les lipides du bol alimentaire et d'autre part sur la sensation de la faim pour limiter la prise de calories sont également particulièrement souhaitables pour aider les personnes en surpoids à retrouver un poids de forme et les personnes soucieuses de leur ligne à éviter une prise de poids tout en leur permettant de continuer à avoir une alimentation équilibrée sans devoir se restreindre. Il serait également souhaitable que cette composition ne présente pas d'effets gênants ou secondaires et qu'elle respecte les directives en vigueur sur les compléments alimentaires.
L'art antérieur connaît un certain nombre de propositions visant à aider les personnes en surpoids à perdre du poids corporel. Il peut être cité la demande WO2000044235 (AJANTA PHARMA Limited) qui propose un produit de carotte pauvre en lipides comprenant 20-50% en poids de fibres de carotte, la teneur en fibres insolubles étant de 15-40% en poids, et la teneur en fibres solubles étant de 5-15%. Ce produit de carotte comprend en outre 10-55% en poids d'hydrates de carbone, 0,02-1% en poids de caroténoïdes et de vitamines et 5-10% en poids d'oligoéléments. Un tel produit de carotte peut être administré par voie orale, notamment pour prévenir et traiter des maladies telles que l'obésité, et les maladies des yeux. Une telle proposition n'apparaît pas de nature à répondre positivement aux objectifs mentionnés précédemment en raison du fait qu'elle ne vise qu'un traitement basé sur l'aspect satiétogène. Ce produit de carotte est en outre riche en hydrates de carbone et ne permet d'induire qu'une modeste perte de poids chez des personnes obèses.
Il convient de citer également le brevet FR2894777 (Laboratoires FORTE PHARMA-MC) qui propose une composition nutraceutique à base de fibres de cacao et de fibres d'oranges. Une telle composition nutraceutique vise uniquement à capter les graisses alimentaires. En outre, il n'est pas démontré que cette composition nutraceutique permette d'induire une perte satisfaisante du poids corporel, en particulier de la masse grasse.
On connaît également par le brevet US2001/012534 (BIYANI MILIND KESHARLAL), un produit de carotte comprenant 20-50% en poids de fibres de carotte, la teneur en fibres insolubles étant de 15-40% en poids, et la teneur en fibres solubles étant de 5-10%, 10-55% en poids d'hydrates de carbone, 0,02- 1 % en poids de caroténoïdes et de vitamines et 5-10% en poids de minéraux et d'oligoéléments. Un tel produit de carotte peut être administré par voie orale, notamment pour prévenir et traiter des maladies telles que l'obésité, et les maladies des yeux. L'administration par voie orale d'un tel produit permet d'améliorer la sensation de satiété et d'induire une perte de poids limitée à 2 à 3 kg chez des personnes obèses sur une période de 2 mois (voir exemple 18). Cette faible perte de poids provient vraisemblablement du fait que les lipides apportés par l'alimentation ne sont pas efficacement captés, mais au contraire massivement absorbés et stockés dans l'organisme.
L'article scientifique de CHOU SY et al., intitulé « Particle Size Reduction Effectively Enhances the Cholesterol-Lowering Activities of Carrot Insoluble Fiber and Cellulose » (J. Agric. Food Chem., vol. 56, n°22, 2008, pages 10994 - 10998), enseigne que la micronisation améliore la capacité de l'extrait de carotte (IFF) à abaisser le taux de lipides sanguins.

Face à cet état des choses, l'objectif principal de l'invention est de proposer une composition nutraceutique qui permette de limiter efficacement l'absorption de lipides alimentaires et d'induire une perte significative de poids chez le mammifère, humain ou animal.

Un autre objectif de l'invention est de fournir une composition permettant de capter efficacement les lipides apportés par l'alimentation, limitant ainsi leur absorption et leur stockage dans l'organisme, et d'induire une perte satisfaisante de poids chez un mammifère, humain ou animal, en particulier chez un sujet humain ayant un IMC compris entre environ 25 et environ 30.

Un autre objectif de l'invention est de proposer une composition nutraceutique dont l'agent actif est particulièrement simple à obtenir.

### Divulgation de l'invention.

La solution proposée par l'invention est une composition nutraceutique pour son utilisation pour limiter l'absorption de lipides alimentaires et pour induire une perte de poids chez un mammifère, humain ou animal, ladite composition nutraceutique étant conforme à la revendication 1.

La demanderesse a mis en évidence qu'un extrait de carotte permet de capter efficacement les lipides apportés par l'alimentation, limitant ainsi leur absorption et leur stockage dans l'organisme et contribuant ainsi à lutter contre la prise de poids indésirable et à induire une perte satisfaisante de poids chez un mammifère, humain ou animal, en particulier chez un sujet humain ayant un IMC compris entre environ 25 et environ 30.

Ces résultats sont d'autant plus surprenants qu'il n'existe aucune donnée, plus particulièrement, *in vivo,* connue à ce jour démontrant l'efficacité des fibres de carotte à limiter l'absorption de lipides alimentaires et à induire une perte satisfaisante du poids corporel.

De façon préférée, la composition nutraceutique pour son utilisation selon l'invention comprend en outre de 2% à 30% p/p$_{composition}$ d'un extrait végétal contenant un inhibiteur de lipase et/ou d'$\alpha$-amylase et/ou d'$\alpha$-D-glucosidase.

Préférentiellement, l'extrait végétal contenant un inhibiteur de lipase et/ou d'$\alpha$-amylase et/ou d'$\alpha$-D-glucosidase est avantageusement choisi dans le groupe suivant : extrait d'algue brune, extrait de haricot blanc, extrait de *Cassia nomame,* extrait du champignon *Phellinus linteus,* extrait de thé vert, extrait de thé noir, un extrait de kiwi, extrait de lichen *Umbilicaria esculenta,* extrait de la plante vietnamienne *Cleistocalyx operculatus,* extrait de la momordique *Mormodica charantia,* extrait de goyave (feuilles) ou autres.

Plus préférentiellement, l'extrait végétal contenant un inhibiteur de lipase et/ou d'$\alpha$-amylase et/ou d'$\alpha$-D-glucosidase est un extrait d'algue brune *Ascophyllum nodosum.*

La composition nutraceutique pour son utilisation selon l'invention contient une quantité inférieure ou égale à 10% p/p$_{composition}$ de fibres alimentaires additionnelles, notamment pour enrichir ladite composition en fibres insolubles en vue de renforcer son action contre l'absorption de calories globales, en particulier de lipides alimentaires en vue d'induire une perte de poids, mais aussi pour normaliser les fonctions du côlon, régulariser le transit-intestinal et lutter contre la tendance à la constipation.

Préférentiellement, ces fibres alimentaires additionnelles sont constituées majoritairement de fibres insolubles et peuvent être obtenues à partir de plantes, de fruits, de légumes, de céréales, de racines ou de leurs mélanges.

Avantageusement, les fibres alimentaires additionnelles sont obtenues à partir de pomme et/ou d'avoine.

La composition nutraceutique pour son utilisation selon l'invention peut en outre comprendre une quantité inférieure ou égale à 5% p/p$_{composition}$ d'un extrait sec de pomme, notamment pour enrichir la composition en composés polyphénoliques, dont la phloridzine, qui sont connus pour moduler la glycémie post-prandiale et pour réguler l'absorption du cholestérol, et ce dans le but de renforcer l'action de la composition visant à limiter l'absorption de lipides alimentaires et à réduire le stockage des graisses.

La composition nutraceutique pour son utilisation selon l'invention peut en outre comprendre une quantité inférieure ou égale à 10% p/p$_{composition}$, de pectine de pomme, notamment pour contribuer à modérer l'appétit et à réguler le transit intestinal. De préférence la pectine de pomme est présente dans la composition nutraceutique pour son utilisation selon l'invention à une teneur comprise entre 4% et 9% P/p$_{composition}$.

De préférence, la composition nutraceutique pour son utilisation comprend :

- de 17 % à 20 % p/p$_{composition}$ d'extrait d'algue brune, de préférence environ 17,3 % p/p$_{composition}$,
- de 45 % à 55 % p/p$_{composition}$ d'extrait de carotte, de préférence environ 48 % p/p$_{composition}$,
- de 3 % à 4 % p/p$_{composition}$ de fibres de pomme, de préférence environ 3,5 % p/p$_{composition}$,
- de 3 % à 4 % p/p$_{composition}$ de fibres d'avoine, de préférence environ 3,5 % p/p$_{composition}$,
- de 2 % à 3 % p/p$_{composition}$ d'extrait de pomme, de préférence environ 2,5 % p/p$_{composition}$, et,
- excipients qsp 100%

La composition nutraceutique pour son utilisation selon l'invention est préférentiellement formulée pour une administration par voie orale, et se présente par exemple, sous forme de poudres, comprimés sécables ou non, pelliculés ou non, de granules, de capsules, de gélules.

La composition nutraceutique pour son utilisation selon l'invention peut être fournie en tant que complément alimentaire, ou pour enrichir une boisson ou un produit alimentaire.

Un autre aspect de l'invention concerne une méthode non thérapeutique pour limiter l'absorption de calories, en particulier de lipides alimentaires et pour induire une perte de poids, ladite méthode non thérapeutique étant conforme à la revendication 8.

D'autres buts et avantages de la présente invention apparaîtront au cours de la description qui va suivre qui n'est donnée qu'à titre indicatif et qui n'a pas pour but de la limiter.

## Modes de réalisation de l'invention.

La composition nutraceutique objet de l'invention est utilisable chez un mammifère, humain ou animal, pour limiter l'absorption de lipides alimentaires et pour induire une perte de poids. Elle est utilisée chez le sujet désirant perdre une portion du poids corporel et de la masse grasse pour améliorer sa santé ou en quête de minceur pour son bien-être physique. Et en particulier, mais non exclusivement, un sujet ayant un IMC compris entre environ 25 et environ 30.

Conformément à l'invention, l'extrait de carotte est utilisé comme agent actif pour limiter l'absorption de lipides alimentaires et pour induire une perte de poids. Dans le contexte de l'invention, on entend par « perte de poids » la perte d'une portion du poids total chez un mammifère, humain ou animal, en particulier la perte d'une portion de la masse grasse.

La carotte, ou *Daucus carota var. sativus,* appartient à la famille des Apiacées. En pratique, on utilise un extrait sec de carotte qui est obtenu à partir d'une purée de carotte (*Daucus carota*) dont le jus est totalement séparé par des techniques classiques d'extraction et de séparation connus de l'homme de l'art. Un tel extrait contient des fibres solubles (pectines) et des fibres insolubles (cellulose et hémicellulose), des minéraux et une faible fraction de lipides et de protéines.

La composition de l'invention comporte au moins 20% p/p$_{composition}$, de préférence au moins 40% p/p$_{composition}$ de l'extrait de carotte.

L'extrait de carotte utilisé a préférentiellement une teneur en fibres totales (fibres solubles et insolubles) comprise entre 50% et 99%, avantageusement entre 60% et 95%, préférentiellement d'environ 92%, en poids rapporté au poids total sec de l'extrait de carotte. La teneur en fibres totales étant déterminée selon la méthode officielle N°56-20 modifiée de l'AACC (Association Americaine des Chimistes des céréales). L'extrait de carotte utilisé est majoritairement constitué de fibres insolubles, le rapport pondéral entre fibres insolubles et fibres solubles étant compris entre 1,02 et 20, avantageusement entre 1,5 et 10, préférentiellement d'environ 6.

L'extrait de carotte est avantageusement utilisé sous forme de poudre fine dont environ 20% des particules présentent un diamètre inférieur à 40 $\mu$m et environ 98% des particules présentent un diamètre inférieur à 100 $\mu$m. On utilise par exemple un extrait de carotte commercialisé par la société ID FOOD sous la dénomination ID 809®.

Un tel extrait de carotte a une capacité de rétention de graisses comprise entre 300% et 500% et un haut pouvoir d'absorption d'eau compris entre 1500 et 3000%, par exemple d'environ 1800%. La capacité de rétention de graisses et le pouvoir d'absorption sont déterminés par la méthode N° 56-20 modifié de l'AACC. L'extrait de carotte confère donc à la composition nutraceutique la faculté de capturer efficacement les lipides alimentaires le long de leur trajet dans l'estomac et l'intestin limitant ainsi de manière optimale l'apport calorique des aliments ingérés.

La composition nutraceutique peut en outre comprendre, un extrait végétal contenant un inhibiteur de lipase et/ou d'$\alpha$-amylase et/ou d'$\alpha$-D-glucosidase, notamment pour limiter l'assimilation des graisses et/ou des sucres du bol alimentaire et diminuer l'accumulation de graisses dans l'organisme en vue de lutter contre la prise globale de calories et d'induire une perte de poids. Pour optimiser cet effet, la teneur de cet extrait végétal est comprise entre 2% et 30% p/p$_{composition}$.

Dans le contexte de la présente invention, on entend par :

- « inhibiteur de lipase » : un composé apte à inhiber l'action des lipases gastriques et pancréatiques responsables du métabolisme et de l'absorption des graisses.
- « inhibiteur d'$\alpha$-amylase » : un composé apte à inhiber l'action de l'$\alpha$-amylase qui est impliquée dans la digestion des glucides, par exemple de l'$\alpha$-amylase sécrétées par les glandes salivaires et par le pancréas.
- « inhibiteur d'$\alpha$-D-glucosidase » un composé apte à inhiber l'action de l'$\alpha$-D-glucosidase qui hydrolyse les disaccharides et/ou les polysaccharides reliés par une liaison $\alpha$-(1,4) pour libérer par exemple le glucose.

A titre d'exemple d'extrait végétal contenant un inhibiteur de lipase et/ou d'$\alpha$-amylase et/ou d'$\alpha$-D-glucosidase on peut citer par exemple un extrait d'algue brune, extrait de haricot blanc, extrait de *Cassia nomame,* extrait du champignon *Phellinus linteus,* extrait de thé vert, extrait de thé noir, un extrait de kiwi, extrait de lichen *Umbilicaria esculenta,* extrait de la plante vietnamienne *Cleistocalyx operculatus,* extrait de la momordique *Mormodica charantia,* extrait de goyave (feuilles) ou autres.

La demanderesse a constaté de manière surprenante qu'un extrait d'algue brune *Ascophyllum nodosum* potentialise l'effet de l'extrait de carotte.

L'*Ascophyllum nodosum ou Ascophylle noueuse* est une algue marine classée dans la famille des Fucacées. L'extrait d'algue brune *Ascophyllum nodosum* utilisé dans la présente invention peut être préparé par tout procédé d'extraction et de purification connu de l'Homme de l'art. Il peut aussi être obtenu auprès des Laboratoires Bio Serae® SAS sous la dénomination commerciale l'ID-alG®.

Dans le contexte de l'invention, il a été découvert que l'association de l'extrait de carotte et de l'extrait d'algue brune *Ascophyllum nodosum* présente un effet complémentaire et synergique pour agir sur l'absorption de lipides alimentaires et de glucides et pour induire une perte significative de poids. Il a en particulier été démontré que cette nouvelle association permet une action à trois niveaux, à savoir une captation des lipides alimentaires, une inhibition des enzymes digestives impliquées dans l'assimilation des graisses et des sucres et une induction de la sensation de satiété.

La composition nutraceutique comprend en outre des fibres alimentaires additionnelles, notamment pour enrichir ladite composition en fibres insolubles en vue de renforcer son action contre l'absorption de calories globales, en particulier de lipides alimentaires, en vue d'induire une perte de poids, mais aussi pour normaliser les fonctions du côlon, régulariser le transit-intestinal et lutter contre la tendance à la constipation. Les fibres alimentaires additionnelles utilisées sont constituées majoritairement de fibres insolubles et sont présentes dans la composition selon l'invention à une teneur inférieure à 10% p/p$_{composition}$. De telles fibres alimentaires additionnelles, peuvent par exemple être obtenues à partir de : plantes telles que le thé vert, thé noir, tige de rhubarbe, cacao, ou autres ; légumineuses telles que les lentilles, haricots blancs ou noirs, fèves, pois chiche, ou autres ; fruits tels que les citrus, framboises, pommes ou autres ; légumes tels que le nopal, céleri, haricots verts, carottes, tomates (peaux), pommes de terre (peaux) ou autres ; céréales telles que le blé, avoine, maïs, graines de lin ou autres ; ou, leurs mélanges.

On préfère toutefois utiliser des fibres alimentaires additionnelles obtenues à partir de pomme et/ou d'avoine, notamment pour l'apport de la composition en fibres insolubles et pour optimiser l'action de la composition visant à limiter l'absorption de calories globales, en particulier de lipides alimentaires et à induire une perte du poids corporel, mais aussi pour normaliser les fonctions du côlon, régulariser le transit intestinal et lutter contre la tendance à la constipation.

Les fibres de pomme utilisées sont issues de pomme (*Pyrus malus*) après séparation du jus par des techniques classiques d'extraction et de séparation connues de l'homme de l'art. Elles présentent une teneur totale en fibres (fibres solubles et insolubles) d'environ 63% en poids rapporté au poids total de la matière sèche. Les fibres insolubles et les fibres solubles représentant respectivement environ 67% et 33% en poids rapporté au poids total de la matière sèche. Les fibres de pomme sont avantageusement utilisées dans la présente invention sous forme de poudre fine dont environ 98% des particules présentent un diamètre inférieur à 315 $\mu$m et 60% des particules présentent un diamètre inférieur à 150 $\mu$m. Les fibres de pomme peuvent être présentes dans la composition nutraceutique à une teneur inférieure ou égale à 10% p/p$_{composition}$, avantageusement à une teneur allant de 3 à 4 % p/p$_{composition}$, de préférence à une teneur d'environ 3,5 % p/p$_{composition}$. Elles sont par exemple vendues sous la dénomination commerciale ID 75® par la Société ID FOOD®.

Les fibres d'avoine utilisées peuvent être obtenues selon toute méthode classique d'extraction et de purification connue de l'homme de l'art à partir d'enveloppes de grains d'avoine (*Avena sativa*). Préférentiellement, les fibres d'avoine présentent une teneur totale en fibres (fibres solubles et insolubles) d'environ 96% en poids rapporté au poids total de la matière sèche et une teneur en eau inférieur à 8%, en poids rapporté au poids total de la matière sèche. Les fibres insolubles et les fibres solubles représentant respectivement environ 93% et 3% en poids rapporté au poids total de la matière sèche. Les fibres d'avoine sont utilisées dans la présente invention sous forme de poudre fine dont au maximum 14% des particules présentent un diamètre supérieur à 32 $\mu$m et au maximum 0,5% des particules présentent un diamètre supérieur à 71 $\mu$m. Les fibres d'avoine peuvent être présentes dans la composition nutraceutique à une teneur inférieure ou égale à 10% p/p$_{composition}$, avantageusement à une teneur allant de 3 à 4 % p/p$_{composition}$, de préférence à une teneur d'environ 3,5 p/p$_{composition}$. Elles sont par exemple vendues sous la dénomination commerciale ID 914® et peuvent être obtenues auprès de la Société ID FOOD®.

Un extrait de pomme peut en outre être utilisé pour enrichir la composition en composés polyphénoliques, dont la phloridzine, qui permettent de moduler la glycémie post-prandiale et pour réguler l'absorption du cholestérol, en vue de réduire le stockage des graisses. Cet extrait de pomme est de préférence présent dans la composition à une teneur inférieure ou égale à 5% p/p$_{composition}$. Un tel extrait de pomme est par exemple le Nutricible® Pomme PHZ qui est fabriqué et commercialisé en France par la société LabAttitude®.

La composition peut également contenir de la pectine de pomme, notamment pour contribuer à gérer la prise alimentaire, en particulier à modérer l'appétit, à ralentir l'absorption des lipides et des glucides. Cette pectine de pomme agit en synergie avec l'extrait de carotte pour induire une perte de poids et favoriser le transit intestinal. La pectine de pomme utilisée est une pectine hautement méthylée présentant de préférence un degré d'estérification compris entre 56 - 63%. Une telle pectine de pomme est composée principalement d'esters méthyliques partiels de l'acide polygalacturonique et de leurs sels d'ammonium, de sodium, de potassium, et/ou de calcium. La pectine de pomme est présente dans la composition à une teneur inférieure ou égale à 10% p/p$_{composition}$, de préférence entre 4% et 9% p/p$_{composition}$. Elle est par exemple vendue sous la dénomination commerciale AF 501® par la Société allemande Herbstreith-Fox®.

Par ailleurs, la composition de l'invention peut en outre contenir des substances capables de faciliter la destruction des espèces oxygénées réactives comme les vitamines E et C, des caroténoïdes, des micronutriments minéraux comme le sélénium et le zinc.

Avantageusement, la composition de l'invention peut comprendre en outre tout excipient approprié, acceptable du point de vue nutraceutique, et connu de l'homme du métier. A titres d'excipients, on peut citer par exemple le phosphate dicalcique, l'alginate de calcium, la carbonate de magnésium, le talc, le stéarate de magnésium, le dioxyde de silicium le chlorure de calcium, ou autres.

Une composition préférée de l'invention comprend :

- de 17 % à 20 % p/p$_{composition}$ d'extrait d'algue brune, de préférence environ 17,3 % p/p$_{composition}$,
- de 45 % à 55 % p/p$_{composition}$ d'extrait de carotte, de préférence environ 48 % p/p$_{composition}$,
- de 3 % à 4 % p/p$_{composition}$ de fibres de pomme, de préférence environ 3,5 % p/p$_{composition}$,
- de 3 % à 4 % p/p$_{composition}$ de fibres d'avoine, de préférence environ 3,5 % p/p$_{composition}$,
- de 2 % à 3 % p/p$_{composition}$ d'extrait de pomme, de préférence environ 2,5 % p/p$_{composition}$, et
- excipients qsp 100%

La composition nutraceutique est administrable par voie orale, par exemple, sous forme de comprimés sécables ou non, pelliculés ou non, de granules, de capsules, de gélules, ou sous forme de poudre libre conditionnée de préférence en sachets unitaires, ou de poudre compressée.

Pour un traitement non thérapeutique de lutte contre l'absorption de lipides et d'entrainement de perte de poids (sujet ayant une IMC inférieure ou égal à 30), la composition objet de l'invention peut être administrée à un mammifère, humain ou animal, à une dose journalière de d'environ 300 mg/repas à environ 1600 mg /repas. En pratique, une posologie adaptée pour limiter l'absorption de lipides alimentaires et pour induire une perte de poids est de préférence de 3 à 6 gélules par jour. A titre d'exemple, la composition peut être prise:

- lors d'un programme continu nommé « perte de poids » d'une durée allant de deux à quatre semaines, et à raison de 3 gélules le midi et 3 gélules le soir au cours du repas avec un verre d'eau, ou,
- à titre occasionnel, à raison de 3 gélules au cours d'au moins un repas copieux le midi et/ou le soir, en vue de réduire d'éventuels écarts alimentaires.

Le programme continu « perte de poids » peut éventuellement être prolongé jusqu'à l'obtention de la perte de poids souhaitée, étant donné que le complément alimentaire selon l'invention est composé de produits naturels qui ne présentent aucun effet gênant ou secondaire et qui sont sans danger pour la santé.

La composition objet de l'invention peut également être utilisée lors d'un traitement thérapeutique de lutte contre l'absorption de lipides et d'entrainement de perte de poids, notamment chez des sujets ayant une IMC supérieur à 30. La posologie est alors définie selon le cas d'espèce.

La composition lorsqu'elle se présente sous forme de poudre peut être incorporée dans toutes formes de produits alimentaires enrichis, par exemple des barres alimentaires, des biscuits salés et/ou sucrés ou autres. Elle peut aussi être dispersée dans l'eau, dans des boissons telles que soda ou jus ou dans des produits laitiers ou dérivés du soja, ou autres.

La composition selon l'invention est notamment utilisable en tant que complément alimentaire, présenté sous n'importe quelle forme compatible avec une absorption orale en une ou plusieurs prises journalières. Ce complément alimentaire peut se présenter notamment sous forme de gélules, de capsules, de comprimés, pelliculés ou non, sécables ou non, de granules, ou sous forme de poudre libre, de préférence conditionnée en sachets unitaires, ou de poudre compressée. Par complément alimentaire, on entend un ensemble comestible destiné à compléter l'alimentation courante d'un organisme humain ou animal, en lui fournissant des nutriments ou autres substances ayant pour fonction de pallier les insuffisances d'une alimentation mal équilibrée. Le complément alimentaire, est donc par définition une denrée alimentaire dont le but est de compléter un régime normal qui constitue une source concentrée d'un nutriment ou d'autres substances ayant un effet nutritionnel ou physiologique, seul ou combiné, commercialisé sous forme de dose. Par dose, on entend toutes les formes classiques, à savoir gélule, pastille, comprimé, granules, pilule, et autres formes similaires ainsi que les sachets en poudre.

**Exemples de compositions ou de compléments alimentaires conformes à l'invention**

*Exemple 1: Composition d'un complément alimentaire présenté sous forme de gélules*

On prépare selon les techniques habituelles une gélule dosée à 120 mg d'extrait de carotte, de fibres d'avoine, et de pectine de pomme ayant la composition suivante :

| Ingrédients | Poids (mg) |
|---|---|
| Extrait de carotte | 90,00 |
| Fibres d'avoine | 15,00 |
| Pectine de pomme | 15,00 |
| Phosphate dicalcique | 76,00 |
| Magnésium carbonate | 50,00 |
| Talc | 9,50 |
| Stéarate de magnésium | 4,50 |
| Dioxyde de silicium | 3,00 |

Les différents ingrédients se présentent sous forme de poudres. Ils sont indépendamment pesés puis incorporés les uns aux autres. Cette composition est désignée ci-après par « composition **A** ».

Exemple 2 : *Composition nutraceutique* présentée sous forme de poudre.

On prépare selon les techniques habituelles une composition sous forme de poudre contenant un extrait de carotte, un extrait d'algue brune *Ascophyllum nodosum,* des fibres de pomme, des fibres d'avoine, un extrait de pomme, et ayant la composition suivante :

| Ingrédient | Poids (mg) |
|---|---|
| Extrait de carotte | 166,67 |
| Extrait d'algue brune | 66,50 |
| Fibres d'avoine | 12,50 |
| Fibres de pomme | 12,50 |
| Extrait de pomme | 8,33 |

Les différents ingrédients se présentent sous forme de poudres. Ils sont indépendamment pesés, puis incorporés les uns aux autres. Cette composition est désignée ci-après par « composition **B** ».

Exemple 3 : *Composition d'un autre* complément alimentaire présenté sous forme de gélules

On prépare selon les techniques habituelles une gélule contenant un extrait de carotte, un extrait d'algue brune *Ascophyllum nodosum,* des fibres de pomme, des fibres d'avoine, un extrait de pomme et des excipients et ayant la composition suivante :

| Ingrédients | %, p/p$_{composition}$ |
|---|---|
| Extrait de carotte | 48,97 |
| Extrait d'algue brune | 19,12 |
| Fibres d'avoine | 3,68 |
| Fibres de pomme | 3,68 |
| Extrait de pomme | 2,50 |
| Alginate de Calcium | 14,71 |
| Chlorure de Calcium | 7,35 |

Tous les ingrédients sont incorporés sous forme de poudre. Ils sont indépendamment pesés puis incorporés les uns aux autres.

**Evaluation de l'efficacité in vitro de capture de graisses alimentaires contenues dans un produit gras, par un témoin et par la composition B de l'exemple 2 ci-dessus**

La capacité de la composition **B** à capturer les graisses alimentaires a été évaluée *in vitro* en utilisant la méthode décrite dans la demande de brevet FR2894777 (Forté Pharma).

Le témoin utilisé est le produit CaloriLight® qui est commercialisé par la société Forté Pharma®.

Les produits testés sont le chocolat, les chips et les pistaches : ces produits on été choisis car ils sont riches en calories, en particulier en lipides et représentent des denrées fréquemment consommées.

On exprime la capacité des fibres à fixer les lipides (Cg) par le rapport suivant :

$$Cg = \frac{M1 - M3}{M2}$$

dans lequel,

Cg = quantité de lipides (g) capturée par gramme de la composition **B** ou de témoin.
M1 = masse de lipides introduite
M2 = masse introduite de la composition **B** ou du témoin
M3 = masse de lipides surnageants

Les résultats sont reproduits dans les tableaux 1 à 3 suivants :

*Tableau 1: résultats obtenus pour le chocolat*

| Echantillon | M1 (mg) | M2 (mg) | M3 (mg) | Cg Moyenne |
|---|---|---|---|---|
| témoin | 3104,63 | 270,27 | 673,5 | 8,995 |
| Composition **B** | 3044,84 | 266,62 | 78,08 | 11,144 |

*Tableau 2 : résultats obtenus pour les chips*

| Echantillon | M1 (mg) | M2 (mg) | M3 (mg) | Cg Moyenne |
|---|---|---|---|---|
| témoin | 2997,1 | 276,14 | 1427,87 | 5,684 |
| Composition **B** | 2955,33 | 263,47 | 316.83 | 10,017 |

*Tableau 3: résultats obtenus pour les pistaches*

| Echantillon | M1 (mg) | M2 (mg) | M3 (mg) | Cg Moyenne |
|---|---|---|---|---|
| témoin | 3050,30 | 270,67 | 759,73 | **8,463** |
| Composition **B** | 2999,68 | 262,83 | 71,46 | **11,148** |

La composition **B** possède une capacité de capture des graisses alimentaires supérieure à celle du produit témoin de l'ordre de :

- 24% pour le chocolat
- 76% pour les chips
- 32% pour les pistaches

Le pourcentage d'absorption de lipides par la composition **B** est présenté au tableau 4 ci-dessous. Il est exprimé par la formule suivante :

$$\text{pourcentage d'absorption de lipides} = \frac{M5}{M1} * 100$$

dans laquelle M5 = masse de lipides absorbée par la composition **B** = M1- M3

*Tableau 4 : pourcentage d'absorption des lipides*

| Source de lipides | M1 (mg) | M3 (mg) | M5 (mg) | Pourcentage d'absorption de lipides (%) |
|---|---|---|---|---|
| chocolat | 3044,84 | 78,08 | 2966,76 | **97** |
| chips | 2955,33 | 316.83 | 2638.50 | **89** |
| pistaches | 2999,68 | 71,46 | 2928.22 | **98** |

La composition **B** permet donc d'absorber environ :

- 97% des lipides contenus dans le chocolat
- 89% des lipides contenus dans les chips
- 98% des lipides contenus dans les pistaches

Si l'on considère que la composition **B** correspond au contenu d'une gélule (266,67 mg d'actifs), on peut en déduire qu'une prise de 3 gélules (0,8 g d'actifs) permettrait de capturer environ :

- 8,92 g de lipides apportés par le chocolat, soit un peu plus que la quantité de lipides apportée par 6 carrés de chocolats, étant donné que 100 g de chocolat contiennent environ 28,8 g de lipides,
- 8,01 g de lipides apportés par les chips, soit un peu moins que la quantité apportée par un sachet de 25 g chips, étant donné que 100 g de chips contiennent environ 36 g de lipides.
- 8,91 g de lipides apportés par environ 16,5 g de pistaches, étant donné que 100 g de pistaches contiennent environ 54 g de lipides.

**Etude clinique**

L'efficacité et la tolérance de la composition **A** décrite dans l'exemple 1, a été étudiée sur des volontaires présentant une surcharge pondérale. Un essai clinique ouvert a été réalisé sur 25 volontaires de sexe féminin et masculin, d'un âge compris entre 18 et 65 ans et répondant aux critères d'une pré-obésité c'est-à-dire présentant un Indice de Masse Corporelle (IMC) supérieur ou égal à 25 et inférieur à 30, et ceci pendant une durée de 15 et 30 jours consécutifs.

Les volontaires de sexe féminin et masculin se sont présentés à 3 reprises à J0, J15 et J30. Lors de J0 les volontaires sont pesés, mesurés afin de vérifier l'IMC. Le périmètre de la cuisse droite, le tour de taille et le tour de hanche ont également été relevés. A J15 et J30, une nouvelle pesée et des mesures des différents périmètres sont alors réalisées.

Il a en outre été demandé aux volontaires d'évaluer leur sensation de satiété sur une période donnée.

La posologie est de 3 gélules le midi et 3 gélules le soir, au début du repas avec un demi-verre d'eau.

Des conseils alimentaires ont été proposés aux volontaires (maintien d'une alimentation équilibrée en qualité et quantité) afin de maîtriser les conditions de l'étude.

Il résulte de cette étude clinique que 100% des volontaires ont perdu du poids de façon homogène. Une perte moyenne de poids de -3,19 Kg (4,26%) a été obtenue sur une durée de 15 jours, avec une perte maximum de -8,4 kg pour la même durée, et une perte moyenne de poids de -6,03 kg (8,04%) a été obtenue sur 30 jours, avec une perte maximum de -10,7 kg pour la même durée. La différence entre J30 et J0 étant statistiquement significative (p<0.0001).

La perte de poids est obtenue uniquement au niveau de la masse grasse, puisqu'une perte moyenne de -5,8 Kg a été obtenue sur la masse grasse (significatif entre J0 et J30, p<.0001), la perte de masse maigre représentant moins de 0,5% du poids total perdu en 30 jours. Soit en moyenne une perte de poids total d'environ 1,5 kg/semaine et de masse grasse d'environ 1,45 kg/semaine.

Le tour de taille a été mesuré à l'aide d'un ruban périmétrique. Une diminution périmétrique a été observée chez 100% des volontaires. La perte moyenne du tour de taille obtenue est de -2,68 cm (2,86%) sur 15 jours et de-4,81 cm (4.81%) sur 30 jours, la perte maximum obtenue étant de -10 cm en 30 jours. Soit en moyenne une perte du tour de taille d'au moins 1,2 cm/semaine.

La perte de poids est évidemment répercutée sur l'indice de masse corporelle : La perte moyenne a été de -

$2.20\ kg/m^2$ (7.93%), avec une perte maximum de $-3.9\ kg/m^2$ sur 30 jours. La différence entre J30 et J0 est significative ($p<0.0001$).

Les résultats montrent que 100% des volontaires ont perdu en moyenne environ 1,5 kg du poids total dès la première semaine et ont sensiblement perdu en volume, en particulier au niveau du périmètre de la taille où le résultat est maximum jusqu'à -10 cm en 30 jours. La perte de poids a été obtenue uniquement au niveau de la masse grasse.

Par ailleurs, la composition évaluée n'a pas présenté d'effets gênants ou secondaires.

En outre, plus de 90% des volontaires ont affirmé que la composition A leur a procuré une sensation de satiété très forte ainsi qu'une sensation de bien-être général.

## Revendications

1. Composition nutraceutique pour son utilisation pour limiter l'absorption de lipides alimentaires en vue d'induire une perte de poids chez un mammifère, humain ou animal, désirant perdre une portion du poids corporel et de la masse grasse pour améliorer sa santé, ladite composition comprenant comme agent actif au moins :

   - un extrait de carotte contenant des fibres solubles et des fibres insolubles, cet extrait de carotte contenant majoritairement des fibres insolubles, la teneur en fibres totales étant comprise entre 60% et 95% en poids rapporté au poids total sec de l'extrait de carotte, le rapport pondéral entre fibres insolubles et fibres solubles étant compris entre 1,02 et 20, préférentiellement entre 1,5 et 10 ; et
   - des fibres alimentaires additionnelles constituées majoritairement de fibres insolubles et obtenues à partir de pomme et/ou d'avoine,

   la teneur en ledit extrait de carotte étant d'au moins 20% $p/p_{composition}$, et la teneur en lesdites fibres alimentaires additionnelles étant inférieure ou égale à 10% $p/p_{composition}$,
   ladite composition étant **caractérisée en ce qu'**elle est administrée par voie orale audit mammifère, humain ou animal, à raison d'une dose de 300 mg/repas à 1600 mg/repas.

2. Composition nutraceutique pour son utilisation selon la revendication 1, **caractérisée en ce qu'**elle comprend en outre de 2% à 30% $p/p_{composition}$ d'un extrait végétal contenant un inhibiteur de lipase et/ou d'$\alpha$-amylase et/ou d'$\alpha$-D-glucosidase, préférablement où l'extrait végétal contenant un inhibiteur de lipase et/ou d'$\alpha$-amylase et/ou d'$\alpha$-D-glucosidase est choisi dans le groupe comprenant un extrait d'algue brune, extrait de haricot blanc, extrait de *Cassia nomame,* extrait du champignon *Phellinus linteus,* extrait de thé vert, extrait de thé noir, un extrait de kiwi, extrait de lichen *Umbilicaria esculenta,* extrait de la plante vietnamienne *Cleistocalyx operculatus,* extrait de la momordique *Mormodica charantia,* extrait de goyave, et plus préférablement où l'extrait végétal contenant un inhibiteur de lipase et/ou d'$\alpha$-amylase et/ou d'$\alpha$-D-glucosidase est un extrait d'algue brune *Ascophyllum nodosum.*

3. Composition nutraceutique pour son utilisation selon l'une des revendications précédentes, **caractérisée en ce qu'**elle comprend en outre un extrait sec de pomme à une teneur inférieure ou égale à 5% $p/p_{composition}$.

4. Composition nutraceutique pour son utilisation selon l'une des revendications précédentes, **caractérisée en ce qu'**elle comprend en outre la pectine de pomme à une teneur inférieure ou égale à 10% $p/p_{composition}$.

5. Composition nutraceutique pour son utilisation selon l'une des revendications précédentes, **caractérisée en ce qu'**elle comprend :

   - de 17 % à 20 % $p/p_{composition}$ d'extrait d'algue brune, de préférence environ 17,3 % $p/p_{composition}$,
   - de 45 % à 55 % $p/p_{composition}$ d'extrait de carotte, de préférence environ 48 % $p/p_{composition}$,
   - de 3 % à 4 % $p/p_{composition}$ de fibres de pomme, de préférence environ 3,5 % $p/p_{composition}$,
   - de 3 % à 4 % $p/p_{composition}$ de fibres d'avoine, de préférence environ 3,5 % $p/p_{composition}$,
   - de 2 % à 3 % $p/p_{composition}$ d'extrait sec de pomme, de préférence environ 2,5 % $p/p_{composition}$, et,
   - excipients qsp 100%.

6. Composition nutraceutique pour son utilisation selon l'une des revendications précédentes, **caractérisée en ce qu'**elle est fournie sous forme de poudre, de comprimés sécables ou non, pelliculés ou non, de granules, de capsules ou de gélules, ou qu'elle est fournie comme un complément alimentaire ou sous forme d'une boisson ou d'un produit alimentaire enrichi en ladite composition nutraceutique.

**7.** Composition nutraceutique pour son utilisation selon la revendication 1, **caractérisée en ce qu'**elle est fournie sous forme de gélules ayant la composition suivante : 90,00 mg d'extrait de carotte ; 15,00 mg de fibres d'avoine ; 15,00 mg de pectine de pomme ; 76,00 mg de phosphate dicalcique ; 50,00 mg de carbonate de magnésium ; 9,50 mg de talc ; 4,50 mg de stéarate de magnésium ; et 3,00 mg de dioxyde de silicium.

**8.** Composition nutraceutique pour son utilisation selon la revendication 1, **caractérisée en outre en ce que** la composition nutraceutique est en outre prévue pour normaliser les fonctions du côlon, régulariser le transit-intestinal et lutter contre la tendance à la constipation.

**9.** Méthode non thérapeutique pour limiter l'absorption de calories en vue d'induire une perte de poids, **caractérisée en ce qu'**une composition nutraceutique comprenant comme agent actif au moins :

- un extrait de carotte contenant des fibres solubles et des fibres insolubles, cet extrait de carotte contenant majoritairement des fibres insolubles, la teneur en fibres totales étant comprise entre 60% et 95% en poids rapporté au poids total sec de l'extrait de carotte, le rapport pondéral entre fibres insolubles et fibres solubles étant compris entre 1,02 et 20, préférentiellement entre 1,5 et 10 ; et
- des fibres alimentaires additionnelles constituées majoritairement de fibres insolubles et obtenues à partir de pomme et/ou d'avoine, la teneur en ledit extrait de carotte étant d'au moins 20% $p/p_{composition}$, et la teneur en lesdites fibres alimentaires additionnelles étant inférieure ou égale à 10% $p/p_{composition}$, est administrée à un mammifère, humain ou animal, en quête de minceur pour son bien-être physique, à raison d'une dose journalière d'environ 300 mg/repas à environ 1600 mg/repas.

**10.** Méthode non thérapeutique selon la revendication 9, **caractérisée en ce que** la composition nutraceutique comprend en outre de 2% à 30% $p/p_{composition}$ d'un extrait végétal contenant un inhibiteur de lipase et/ou d'$\alpha$-amylase et/ou d'$\alpha$-D-glucosidase, préférablement où l'extrait végétal contenant un inhibiteur de lipase et/ou d'$\alpha$-amylase et/ou d'$\alpha$-D-glucosidase est choisi dans le groupe comprenant un extrait d'algue brune, extrait de haricot blanc, extrait de *Cassia nomame,* extrait du champignon *Phellinus linteus,* extrait de thé vert, extrait de thé noir, un extrait de kiwi, extrait de lichen *Umbilicaria esculenta,* extrait de la plante vietnamienne *Cleistocalyx operculatus,* extrait de la momordique *Mormodica charantia,* extrait de goyave, et plus préférablement où l'extrait végétal contenant un inhibiteur de lipase et/ou d'$\alpha$-amylase et/ou d'$\alpha$-D-glucosidase est un extrait d'algue brune *Ascophyllum nodosum.*

**11.** Méthode non thérapeutique selon l'une des revendications 9 ou 10, **caractérisée en ce que** la composition nutraceutique comprend en outre un extrait sec de pomme à une teneur inférieure ou égale à 5% $p/p_{composition}$.

**12.** Méthode non thérapeutique selon l'une des revendications 9, 10 ou 11, **caractérisée en ce que** la composition nutraceutique comprend en outre la pectine de pomme à une teneur inférieure ou égale à 10% $p/p_{composition}$.

**13.** Méthode non thérapeutique selon l'une des revendications 9, 10, 11 ou 12, **caractérisée en ce que** la composition nutraceutique comprend :

- de 17 % à 20 % $p/p_{composition}$ d'extrait d'algue brune, de préférence environ 17,3 % $p/p_{composition}$,
- de 45 % à 55 % $p/p_{composition}$ d'extrait de carotte, de préférence environ 48 % $p/p_{composition}$,
- de 3 % à 4 % $p/p_{composition}$ de fibres de pomme, de préférence environ 3,5 % $p/p_{composition}$,
- de 3 % à 4 % $p/p_{composition}$ de fibres d'avoine, de préférence environ 3,5 % $p/p_{composition}$,
- de 2 % à 3 % $p/p_{composition}$ d'extrait sec de pomme, de préférence environ 2,5 % $p/p_{composition}$, et,
- excipients qsp 100%.

**14.** Méthode non thérapeutique selon l'une des revendications 9, 10, 11, 12 ou 13, **caractérisée en ce que** la composition nutraceutique est fournie sous forme de poudre, de comprimés sécables ou non, pelliculés ou non, de granules, de capsules ou de gélules ; ou qu'elle est fournie comme un complément alimentaire ou sous forme d'une boisson ou d'un produit alimentaire enrichi en ladite composition nutraceutique.

**15.** Méthode non thérapeutique selon la revendication 9, **caractérisée en ce que** la composition nutraceutique est fournie sous forme de gélules ayant la composition suivante : 90,00 mg d'extrait de carotte ; 15,00 mg de fibres d'avoine ; 15,00 mg de pectine de pomme ; 76,00 mg de phosphate dicalcique ; 50,00 mg de carbonate de magnésium ; 9,50 mg de talc ; 4,50 mg de stéarate de magnésium ; et 3,00 mg de dioxyde de silicium.

**16.** Méthode non thérapeutique selon l'une des revendications 9, 10, 11, 12, 13, 14 ou 15, **caractérisée en ce que** ledit mammifère est un sujet humain ayant un Indice de Masse Corporel (IMC) qui est compris entre 25 et 30.

**Patentansprüche**

1. Nutrazeutische Zusammensetzung zur Verwendung zum Einschränken der Resorption von Nahrungslipiden für den Gewichtsverlust bei einem menschlichen oder tierischen Säuger, der zur Verbesserung seiner Gesundheit einen Teil des Körpergewichts und der Fettmasse verlieren möchte, wobei die Zusammensetzung als Wirkstoff mindestens Folgendes umfasst:

   - einen Karottenextrakt, der lösliche Ballaststoffe und unlösliche Ballaststoffe enthält, wobei dieser Karottenextrakt hauptsächlich unlösliche Ballaststoffe enthält, wobei der Gesamtballaststoffgehalt zwischen 60 Ges.-% und 95 Ges.-% in Bezug auf das Gesamttrockengewicht des Karottenextrakts beträgt, wobei das Gewichtsverhältnis zwischen unlöslichen Ballaststoffen und löslichen Ballaststoffen zwischen 1,02 und 20, vorzugsweise zwischen 1,5 und 10 beträgt; und
   - zusätzliche Nahrungsballaststoffe, die hauptsächlich aus unlöslichen Ballaststoffen bestehen und aus Apfel und/oder Hafer stammen,

   wobei der Gehalt des Karottenextrakts mindestens 20% w/w$_{Zusammensetzung}$ beträgt und der Gehalt an zusätzlichen Nahrungsballaststoffen 10% w/w$_{Zusammensetzung}$ oder weniger beträgt,
   wobei die Zusammensetzung **dadurch gekennzeichnet ist, dass** sie dem menschlichen oder tierischen Säuger oral in einer Dosis von 300 mg/Mahlzeit bis 1600 mg/Mahlzeit verabreicht wird.

2. Nutrazeutische Zusammensetzung zur Verwendung nach Anspruch 1, **dadurch gekennzeichnet, dass** sie weiterhin 2% w/w$_{Zusammensetzung}$ bis 30% w/w$_{Zusammensetzung}$ eines Pflanzenextrakts, der einen Lipase- und/oder $\alpha$-Amylase- und/oder $\alpha$-D-Glucosidasehemmer enthält, umfasst, vorzugsweise wobei der Pflanzenextrakt, der einen Lipase- und/oder $\alpha$-Amylase- und/oder $\alpha$-D-Glucosidasehemmer enthält, aus der Gruppe bestehend aus einem Braunalgenextrakt, Weißbohnenextrakt, *Cassia-nomame*-Extrakt, Extrakt des Pilzes *Phellinus linteus,* Grünteeextrakt, Schwarzteeextrakt, Kiwiextrakt, Extrakt der Flechte *Umbilicaria esculenta,* Extrakt der vietnamesischen Pflanze *Cleistocalyx operculatus,* Extrakt der Bittermelone *Mormodica charantia,* Goyavaextrakt ausgewählt ist, und besonders bevorzugt wobei der Pflanzenextrakt, der einen Lipase- und/oder $\alpha$-Amylase- und/oder $\alpha$-D-Glucosidasehemmer enthält, ein Extrakt der Braunalge *Ascophyllum nodosum* ist.

3. Nutrazeutische Zusammensetzung zur Verwendung nach einem der vorhergehenden Ansprüche, **dadurch gekennzeichnet, dass** sie weiterhin einen Apfeltrockenextrakt in einem Gehalt von 5% w/w$_{Zusammensetzung}$ oder weniger umfasst.

4. Nutrazeutische Zusammensetzung zur Verwendung nach einem der vorhergehenden Ansprüche, **dadurch gekennzeichnet, dass** sie weiterhin Apfelpektin in einem Gehalt von 10% w/w$_{Zusammensetzung}$ oder weniger umfasst.

5. Nutrazeutische Zusammensetzung zur Verwendung nach einem der vorhergehenden Ansprüche, **dadurch gekennzeichnet, dass** sie Folgendes umfasst:

   - 17% w/w$_{Zusammensetzung}$ bis 20% w/w$_{Zusammensetzung}$ Braunalgenextrakt, vorzugsweise ungefähr 17,3% w/w$_{Zusammensetzung}$,
   - 45% w/w$_{Zusammensetzung}$ bis 55% w/w$_{Zusammensetzung}$ Karottenextrakt, vorzugsweise ungefähr 48% w/w$_{Zusammensetzung}$,
   - 3% w/w$_{Zusammensetzung}$ bis 4% w/w$_{Zusammensetzung}$ Apfelballaststoffe, vorzugsweise ungefähr 3,5% w/w$_{Zusammensetzung}$,
   - 3% w/w$_{Zusammensetzung}$ bis 4% w/w$_{zusammensetzung}$ Haferballaststoffe, vorzugsweise ungefähr 3,5% w/w$_{Zusammensetzung}$,
   - 2% w/w$_{Zusammensetzung}$ bis 3% w/w$_{Zusammensetzung}$ Apfeltrockenextrakt, vorzugsweise ungefähr 2,5% w/w$_{Zusammensetzung}$, sowie
   - Grundstoffe ad 100%.

6. Nutrazeutische Zusammensetzung zur Verwendung nach einem der vorhergehenden Ansprüche, **dadurch gekennzeichnet, dass** sie in Form eines Pulvers, von teilbaren oder nichtteilbaren Tabletten oder Filmtabletten, von Granulatkörnern, von Kapseln oder von Gelkapseln bereitgestellt wird, oder dass sie als Nahrungsmittelergänzungsstoff oder in Form eines an dieser nutrazeutischen Zusammensetzung angereicherten Getränks oder Nahrungsmittelprodukts bereitgestellt wird.

7. Nutrazeutische Zusammensetzung zur Verwendung nach Anspruch 1, **dadurch gekennzeichnet, dass** sie in Form von Gelkapseln mit der folgenden Zusammensetzung bereitgestellt wird: 90,00 mg Karottenextrakt; 15,00 mg Haferballaststoffe; 15,00 mg Apfelpektin; 76,00 mg Dicalciumphosphat; 50,00 mg Magnesiumcarbonat; 9,50 mg Talkum; 4,50 mg Magnesiumstearat; und 3,00 mg Siliziumdioxid.

8. Nutrazeutische Zusammensetzung zur Verwendung nach Anspruch 1, weiter **dadurch gekennzeichnet, dass** die nutrazeutische Zusammensetzung weiterhin für die Normalisierung der Kohlenfunktionen, das Regelmäßigmachen der Darmpassage und die Bekämpfung der Verstopfungsneigung vorgesehen ist.

9. Nichttherapeutisches Verfahren zur Einschränkung der Kalorienaufnahme für den Gewichtsverlust, **dadurch gekennzeichnet, dass** man einem menschlichen oder tierischen Säuger, der für sein körperliches Wohlbefinden abnehmen will, eine nutrazeutische Zusammensetzung, die als Wirkstoff mindestens Folgendes umfasst:

- einen Karottenextrakt, der lösliche Ballaststoffe und unlösliche Ballaststoffe enthält, wobei dieser Karottenextrakt hauptsächlich unlösliche Ballaststoffe enthält, wobei der Gesamtballaststoffgehalt zwischen 60 Gew.-% und 95 Ges.-% in Bezug auf das Gesamttrockengewicht des Karottenextrakts beträgt, wobei das Gewichtsverhältnis zwischen unlöslichen Ballaststoffen und löslichen Ballaststoffen zwischen 1,02 und 20, vorzugsweise zwischen 1,5 und 10 beträgt; und
- zusätzliche Nahrungsballaststoffe, die hauptsächlich aus unlöslichen Ballaststoffen bestehen und aus Apfel und/oder Hafer stammen,

wobei der Gehalt des Karottenextrakts mindestens 20% w/w$_{Zusammensetzung}$ beträgt und der Gehalt an zusätzlichen Nahrungsballaststoffen 10% w/w$_{Zusammensetzung}$ oder weniger beträgt, in einer Tagesdosis von ungefähr 300 mg/Mahlzeit bis ungefähr 1600 mg/Mahlzeit verabreicht.

10. Nichttherapeutisches Verfahren nach Anspruch 9, **dadurch gekennzeichnet, dass** die nutrazeutische Zusammensetzung weiterhin 2% w/w$_{Zusammensetzung}$ bis 30% w/w$_{Zusammensetzung}$ eines Pflanzenextrakts, der einen Lipase- und/oder $\alpha$-Amylase- und/oder $\alpha$-D-Glucosidasehemmer enthält, umfasst, vorzugsweise wobei der Pflanzenextrakt, der einen Lipase- und/oder $\alpha$-Amylase- und/oder $\alpha$-D-Glucosidasehemmer enthält, aus der Gruppe bestehend aus einem Braunalgenextrakt, Weißbohnenextrakt, *Cassia-nomame*-Extrakt, Extrakt des Pilzes *Phellinus linteus,* Grünteeextrakt, Schwarzteeextrakt, Kiwiextrakt, Extrakt der Flechte *Umbilicaria esculenta,* Extrakt der vietnamesischen Pflanze *Cleistocalyx operculatus,* Extrakt der Bittermelone *Mormodica charantia,* Goyavaextrakt ausgewählt ist, und besonders bevorzugt wobei der Pflanzenextrakt, der einen Lipase- und/oder $\alpha$-Amylase- und/oder $\alpha$-D-Glucosidasehemmer enthält, ein Extrakt der Braunalge *Ascophyllum nodosum* ist.

11. Nichttherapeutisches Verfahren nach einem der Ansprüche 9 oder 10, **dadurch gekennzeichnet, dass** die nutrazeutische Zusammensetzung weiterhin einen Apfeltrockenextrakt in einem Gehalt von 5% w/w$_{Zusammensetzung}$ oder weniger umfasst.

12. Nichttherapeutisches Verfahren nach einem der Ansprüche 9, 10 oder 11, **dadurch gekennzeichnet, dass** die nutrazeutische Zusammensetzung weiterhin Apfelpektin in einem Gehalt von 10% w/w$_{Zusammensetzung}$ oder weniger umfasst.

13. Nichttherapeutisches Verfahren nach einem der Ansprüche 9, 10, 11 oder 12, **dadurch gekennzeichnet, dass** die nutrazeutische Zusammensetzung Folgendes umfasst:

- 17% w/w$_{Zusammensetzung}$ bis 20% w/w$_{Zusammensetzung}$ Braunalgenextrakt, vorzugsweise ungefähr 17,3% w/w$_{Zusammensetzung}$,
- 45% w/w$_{Zusammensetzung}$ bis 55% w/w$_{Zusammensetzung}$ Karottenextrakt, vorzugsweise ungefähr 48% w/w$_{Zusammensetzung}$,
- 3% w/w$_{Zusammensetzung}$ bis 4% w/w$_{Zusammensetzung}$ Apfelballaststoffe, vorzugsweise ungefähr 3,5% w/w$_{Zusammensetzung}$,
- 3% w/w$_{Zusammensetzung}$ bis 4% w/w$_{Zusammensetzung}$ Haferballaststoffe, vorzugsweise ungefähr 3,5% w/w$_{Zusammensetzung}$,
- 2% w/w$_{Zusammensetzung}$ bis 3% w/w$_{Zusammensetzung}$ Apfeltrockenextrakt, vorzugsweise ungefähr 2,5% w/w$_{Zusammensetzung}$, sowie
- Grundstoffe ad 100%.

14. Nichttherapeutisches Verfahren nach einem der Ansprüche 9, 10, 11, 12 oder 13, **dadurch gekennzeichnet, dass** die nutrazeutische Zusammensetzung in Form eines Pulvers, von teilbaren oder nichtteilbaren Tabletten oder Filmtabletten, von Granulatkörnern, von Kapseln oder von Gelkapseln bereitgestellt wird, oder dass sie als Nahrungsmittelergänzungsstoff oder in Form eines an dieser nutrazeutischen Zusammensetzung angereicherten Getränks oder Nahrungsmittelprodukts bereitgestellt wird.

15. Nichttherapeutisches Verfahren nach Anspruch 9, **dadurch gekennzeichnet, dass** die nutrazeutische Zusammensetzung in Form von Gelkapseln mit der folgenden Zusammensetzung bereitgestellt wird: 90,00 mg Karottenextrakt; 15,00 mg Haferballaststoffe; 15,00 mg Apfelpektin; 76,00 mg Dicalciumphosphat; 50,00 mg Magnesiumcarbonat; 9,50 mg Talkum; 4,50 mg Magnesiumstearat; und 3,00 mg Siliziumdioxid.

16. Nichttherapeutisches Verfahren nach einem der Ansprüche 9, 10, 11, 12, 13, 14 oder 15, **dadurch gekennzeichnet, dass** es sich bei dem Säuger um ein menschliches Individuum mit einem Körpermasseindex (KMI) zwischen 25 und 30 handelt.

**Claims**

1. Nutraceutical composition for use thereof for limiting the absorption of dietary lipids with a view to inducing weight loss in a human or animal mammal wanting to lose a portion of bodyweight and of body fat in order to improve their health, said composition comprising, as active agent, at least:

   - a carrot extract containing soluble fibres and insoluble fibres, this carrot extract containing predominantly insoluble fibres, the total fibre content being between 60% and 95% by weight relative to the total dry weight of the carrot extract, the weight ratio between insoluble fibres and soluble fibres being between 1.02 and 20, preferentially between 1.5 and 10; and
   - additional dietary fibres consisting predominantly of insoluble fibres and obtained from apple and/or from oats,

   the content of said carrot extract being at least 20% w/w$_{composition}$, and the content of said additional dietary fibres being less than or equal to 10% w/w$_{composition}$,
   said composition being **characterized in that** it is administered orally to said human or animal mammal in a proportion of a dose of 300 mg/meal to 1600 mg/meal.

2. Nutraceutical composition for use thereof according to Claim 1, **characterized in that** it also comprises from 2% to 30% w/w$_{composition}$ of a plant extract containing an inhibitor of lipase and/or of $\alpha$-amylase and/or of $\alpha$-D-glucosidase, preferably wherein the plant extract containing an inhibitor of lipase and/or of $\alpha$-amylase and/or of $\alpha$-D-glucosidase is chosen from the group comprising a brown alga extract, a haricot bean extract, a *Cassia nomame* extract, an extract of the mushroom *Phellinus linteus,* a green tea extract, a black tea extract, a kiwi extract, an extract of the lichen *Umbilicaria esculenta,* an extract of the Vietnamese plant *Cleistocalyx operculatus,* an extract of the bitter melon *Mormodica charantia* and a guava extract, and more preferably wherein the plant extract containing an inhibitor of lipase and/or of $\alpha$-amylase and/or of $\alpha$-D-glucosidase is an extract of the brown alga *Ascophyllum nodosum.*

3. Nutraceutical composition for use thereof according to either of the preceding claims, **characterized in that** it also comprises a dry extract of apple at a content of less than or equal to 5% w/w$_{composition}$.

4. Nutraceutical composition for use thereof according to one of the preceding claims, **characterized in that** it also comprises apple pectin at a content of less than or equal to 10% w/w$_{composition}$.

5. Nutraceutical composition for use thereof according to one of the preceding claims, **characterized in that** it comprises:

   - from 17% to 20% w/w$_{composition}$ of brown alga extract, preferably approximately 17.3% w/w$_{composition}$,
   - from 45% to 55% w/w$_{composition}$ of carrot extract, preferably approximately 48% w/w$_{composition}$,
   - from 3% to 4% w/w$_{composition}$ of apple fibres, preferably approximately 3.5% w/w$_{composition}$,

- from 3% to 4% w/w$_{composition}$ of oat fibres, preferably approximately 3.5% w/w$_{composition}$,
- from 2% to 3% w/w$_{composition}$ of dry extract of apple, preferably approximately 2.5% w/w$_{composition}$, and
- excipients, qs 100%.

6. Nutraceutical composition for use thereof according to one of the preceding claims, **characterized in that** it is provided in the form of a powder, of scored or unscored, film-coated or non-film-coated tablets, of granules, of capsules or of gel capsules, or **in that** it is provided as a food supplement or in the form of a drink or of a food product enriched with said nutraceutical composition.

7. Nutraceutical composition for use thereof according to Claim 1, **characterized in that** it is provided in the form of gel capsules having the following composition: 90.00 mg of carrot extract; 15.00 mg of oat fibres; 15.00 mg of apple pectin; 76.00 mg of dicalcium phosphate; 50.00 mg of magnesium carbonate; 9.50 mg of talc; 4.50 mg of magnesium stearate; and 3.00 mg of silicon dioxide.

8. Nutraceutical composition for use thereof according to Claim 1, **also characterized in that** the nutraceutical composition is also provided for normalizing the colon functions, regulating intestinal transit and combatting a tendency towards constipation.

9. Non-therapeutic method for limiting calorie absorption with a view to inducing weight loss, **characterized in that** a nutraceutical composition comprising, as active agent, at least:

  - a carrot extract containing soluble fibres and insoluble fibres, this carrot extract containing predominantly insoluble fibres, the total fibre content being between 60% and 95% by weight relative to the total dry weight of the carrot extract, the weight ratio between insoluble fibres and soluble fibres being between 1.02 and 20, preferentially between 1.5 and 10; and
  - additional dietary fibres consisting predominantly of insoluble fibres and obtained from apple and/or from oats,

the content of said carrot extract being at least 20% w/w$_{composition}$, and the content of said additional dietary fibres being less than or equal to 10% w/w$_{composition}$,
is administered to a human or animal mammal trying to lose weight for their physical well-being, in a proportion of a daily dose of approximately 300 mg/meal to approximately 1600 mg/meal.

10. Non-therapeutic method according to Claim 9, **characterized in that** the nutraceutical composition also comprises from 2% to 30% w/w$_{composition}$ of a plant extract containing an inhibitor of lipase and/or of α-amylase and/or of α-D-glucosidase, preferably wherein the plant extract containing an inhibitor of lipase and/or of α-amylase and/or of α-D-glucosidase is chosen from the group comprising a brown alga extract, a haricot bean extract, a *Cassia nomame* extract, an extract of the mushroom *Phellinus linteus,* a green tea extract, a black tea extract, a kiwi extract, an extract of the lichen *Umbilicaria esculenta,* an extract of the Vietnamese plant *Cleistocalyx operculatus,* an extract of the bitter melon *Mormodica charantia* and a guava extract, and more preferably wherein the plant extract containing an inhibitor of lipase and/or of α-amylase and/or of α-D-glucosidase is an extract of the brown alga *Ascophyllum nodosum.*

11. Non-therapeutic method according to either of Claims 9 and 10, **characterized in that** the nutraceutical composition also comprises a dry extract of apple at a content of less than or equal to 5% w/w$_{composition}$.

12. Non-therapeutic method according to one of Claims 9, 10 and 11, **characterized in that** the nutraceutical composition also comprises apple pectin at a content of less than or equal to 10% w/w$_{composition}$.

13. Non-therapeutic method according to one of Claims 9, 10, 11 and 12, **characterized in that** the nutraceutical composition comprises:

  - from 17% to 20% w/w$_{composition}$ of brown alga extract, preferably approximately 17.3% w/w$_{composition}$,
  - from 45% to 55% w/w$_{composition}$ of carrot extract, preferably approximately 48% w/w$_{composition}$,
  - from 3% to 4% w/w$_{composition}$ of apple fibres, preferably approximately 3.5% w/w$_{composition}$,
  - from 3% to 4% w/w$_{composition}$ of oat fibres, preferably approximately 3.5% w/w$_{composition}$,
  - from 2% to 3% w/w$_{composition}$ of dry extract of apple, preferably approximately 2.5% w/w$_{composition}$, and
  - excipients, qs 100%.

**14.** Non-therapeutic method according to one of Claims 9, 10, 11, 12 and 13, **characterized in that** the nutraceutical composition is provided in the form of a powder, of scored or unscored, film-coated or non-film-coated tablets, of granules, of capsules or of gel capsules; or **in that** it is provided as a food supplement or in the form of a drink or of a food product enriched with said nutraceutical composition.

**15.** Non-therapeutic method according to Claim 9, **characterized in that** the nutraceutical composition is provided in the form of gel capsules having the following composition: 90.00 mg of carrot extract; 15.00 mg of oat fibres; 15.00 mg of apple pectin; 76.00 mg of dicalcium phosphate; 50.00 mg of magnesium carbonate; 9.50 mg of talc; 4.50 mg of magnesium stearate; and 3.00 mg of silicon dioxide.

**16.** Non-therapeutic method according to one of Claims 9, 10, 11, 12, 13, 14 and 15, **characterized in that** said mammal is a human subject with a Body Mass Index (BMI) which is between 25 and 30.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2000044235 A
- FR 2894777
- US 2001012534 A

**Littérature non-brevet citée dans la description**

- **CHOU SY et al.** Particle Size Reduction Effectively Enhances the Cholesterol-Lowering Activities of Carrot Insoluble Fiber and Cellulose. *J. Agric. Food Chem.,* 2008, vol. 56 (22), 10994-10998